(19) Europäisches Patentamt
European Patent Office
Office européen des brevets

(11) Veröffentlichungsnummer: **0 341 541 B1**

(12) # EUROPÄISCHE PATENTSCHRIFT

(45) Veröffentlichungstag der Patentschrift: **02.02.94**

(51) Int. Cl.[5]: **C09B 57/00**, C07C 271/08, G11B 7/24

(21) Anmeldenummer: **89107888.3**

(22) Anmeldetag: **29.04.89**

(54) **Urethangruppen aufweisende Azulenquadratsäurefarbstoffe, deren Vorprodukte sowie optisches Aufzeichnungsmedium.**

(30) Priorität: **11.05.88 DE 3816068**

(43) Veröffentlichungstag der Anmeldung:
**15.11.89 Patentblatt 89/46**

(45) Bekanntmachung des Hinweises auf die Patenterteilung:
**02.02.94 Patentblatt 94/05**

(84) Benannte Vertragsstaaten:
**BE CH DE ES FR GB IT LI NL**

(56) Entgegenhaltungen:
**EP-A- 0 310 080**

(73) Patentinhaber: **BASF Aktiengesellschaft**
**Carl-Bosch-Strasse 38**
**D-67063 Ludwigshafen(DE)**

(72) Erfinder: **Schrott, Wolfgang, Dr.**
**Bruesseler Ring 45**
**D-6700 Ludwigshafen(DE)**
Erfinder: **Neumann, Peter, Dr.**
**Poststrasse 28**
**D-6800 Mannheim 31(DE)**
Erfinder: **Schmitt, Michael Dr.**
**Sternstrasse 217**
**D-6700 Ludwigshafen(DE)**
Erfinder: **Brosius, Sibylle, Dr.**
**L 9,3,**
**D-6800 Mannheim 1(DE)**
Erfinder: **Schomann, Klaus Dieter, Dr.**
**Kopernikusstrasse 47**
**D-6700 Ludwigshafen(DE)**
Erfinder: **Kuppelmaier, Harald, Dr.**
**Kuehler Grund 30 a**
**D-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 341 541 B1

Rank Xerox (UK) Business Services
(3.10/3.09/3.3.3)

## Beschreibung

Die vorliegende Erfindung betrifft Urethangruppen aufweisende Azulenquadratsäurefarbstoffe der Formel I

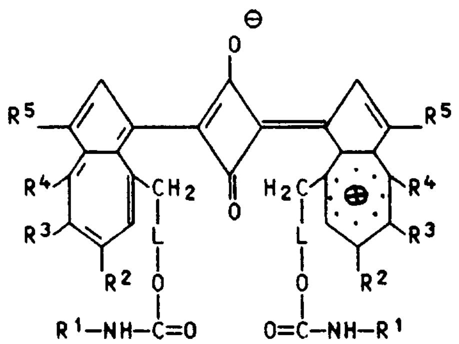

(I),

in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten, |

mit der Maßgabe, daß wenn $R^5$ Wasserstoff bedeutet, an einem oder beiden Azulenringen die Ringpositionen der Substituenten $CH_2$-L-O-CO-NH$R^1$ und $R^4$ innerhalb eines Azulenrings auch gegeneinander vertauscht sein können, deren Vorprodukte sowie ein optisches Aufzeichnungsmedium, das die neuen Farbstoffe enthält.

Zur kostengünstigen Herstellung optischer Datenaufzeichnungsträger werden Farbstoffe mit besonderen Eigenschaften benötigt. Diese Farbstoffe sollten

- eine starke Absorption zwischen 700 und 900 nm aufweisen, um mit Halbleiterlasern beschreibbare Schichten zu liefern,
- in Schicht eine hohe Reflektivität im nahen Infrarot (700 bis 900 nm) aufweisen, um mit einem einfachen Schichtaufbau (ohne Reflektorschicht] auszukommen,
- eine hohe Löslichkeit aufweisen, um beispielsweise die dünne Speicherschicht durch Spincoating auf einen Träger aufbringen zu können, und
- in dünnen Schichten eine hohe Stabilität aufweisen.

Die bisher bekannten Speichermaterialien weisen zumindest in einem der genannten Anforderungspunkte Mängel auf.

Aufgabe der vorliegenden Erfindung war es daher, neue Farbstoffe bereitzustellen, bei denen die obengenannten Mängel nicht mehr oder höchstens nur in äußerst geringem Maße auftreten.

Demgemäß wurden die oben näher bezeichneten Azulenquadratsäurefarbstoffe der Formel I gefunden.

Alle in der obengenannten Formel I auftretenden Alkylen- und Alkylgruppen können sowohl geradkettig als auch verzweigt sein.

Wenn in Formel I substituierte Phenylgruppen auftreten, kommen als Substituenten z.B. $C_1$-$C_4$-Alkyl, $C_1$-$C_4$-Alkoxy oder Halogen in Betracht.

Als Halogen ist jeweils Fluor, Chlor oder Brom bevorzugt.

Reste L sind z.B. Methylen, Ethylen, 1,2- oder 1,3-Propylen, 1,2-, 1,3-, 2,3- oder 1 ,4-Butylen, Pentamethylen, Hexamethylen, Heptamethylen, Octamethylen, Nonamethylen, Decamethylen, Undecamethylen, Dodecamethylen, Phenylethylen, 1-Phenyl-1,2-propylen oder 2-Phenyl-1 ,3-propylen.

Reste $R^1$, $R^2$, $R^3$, $R^4$ und $R^5$ in Formel I sind beispielsweise Methyl, Ethyl, Propyl, Isopropyl, Butyl, Isobutyl, sec-Butyl, tert-Butyl, Pentyl, Isopentyl, Neopentyl, tert-Pentyl, 2-Methylbutyl, Hexyl, 2-Methylpentyl, Heptyl, Octyl, 2-Ethylhexyl, Nonyl, Decyl, Undecyl oder Dodecyl.

Reste $R^1$ sind weiterhin z.B. Tridecyl, Tetradecyl, Pentadecyl, Hexadecyl, Heptadecyl, Octadecyl, Nonadecyl, Eicosyl, Cyclopentyl, Cyclohexyl, Cycloheptyl, Phenyl, 2- oder 4-Methylphenyl, 2- oder 4-Methoxyphenyl, 2- oder 4-Chlorphenyl oder 2,4-Dichlorphenyl.

Reste $R^2$, $R^3$, $R^4$ und $R^5$ sind weiterhin z.B. Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)-ethyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

Bevorzugt sind Azulenquadratsäurefarbstoffe der Formel I, in der $R^2$, $R^3$, $R^4$ und $R^5$ jeweils $C_1$-$C_6$-Alkyl bedeuten.

Weiterhin bevorzugt sind Azulenquadratsäurefarbstoffe der Formel I, in der $R^1$ $C_1$-$C_{18}$-Alkyl oder Cyclohexyl bedeutet.

Besonders bevorzugt sind Azulenquadratsäurefarbstoffe der Formel I, in der $R^2$ und $R^4$ jeweils Methyl und $R^3$ und $R^5$ jeweils Wasserstoff bedeuten und L und $R^1$ jeweils die obengenannte Bedeutung besitzen, Diese Farbstoffe entsprechen der Formel Ia

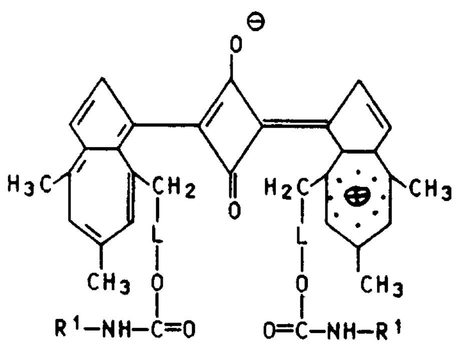

(Ia).

Ganz besonders bevorzugt sind Azulenquadratsäurefarbstoffe der Formel I in der $R^2$ und $R^4$ jeweils Wasserstoff, $R^3$ Isopropyl und $R^5$ Methyl bedeuten und L und $R^1$ jeweils die obengenannte Bedeutung besitzen. Diese Farbstoffe entsprechen der Formel Ib

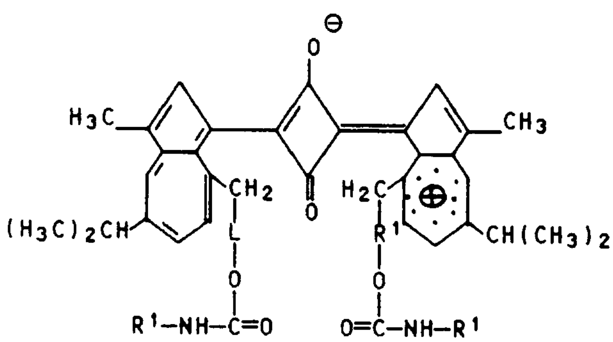

(Ib).

Die Farbstoffe der Formel I werden aus Azulenderivaten der Formel II, in der L, $R^1$, $R^2$, $R^3$, $R^4$ und $R^5$ jeweils die obengenannte Bedeutung besitzen, durch Umsetzung mit Quadratsäure der Formel III gemäß der folgenden Gleichung erhalten:

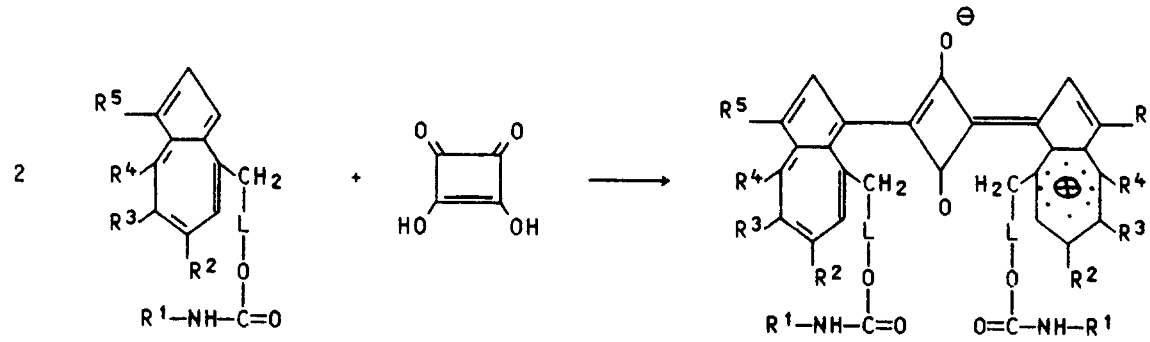

(II) (III) (I)

Bei denjenigen Azulenderivaten der Formel II, in denen $R^5$ Wasserstoff bedeutet, kann die Verknüpfung zur Quadratsäure an unterschiedlichen Ringpositionen des Fünfrings erfolgen, wobei isomere Produkte entstehen können, in denen die Ringpositionen der Substituenten $CH_2$-L-O-CO-NH-$R^1$ und $R^4$, wie oben ausgeführt, gegeneinander vertauscht sind. Es sind nämlich dann Verbindungen, bei denen die Bindung zur Quadratsäure an derjenigen Seite erfolgt, an der der Substituent $CH_2$-L-O-CO-NH-$R^1$ gebunden ist, von solchen Verbindungen zu unterscheiden, bei denen die Bindung zur Quadratsäure an derjenigen Seite erfolgt, an der der Substituent $R^4$ gebunden ist. Diese isomeren Verbindungen können chromatographisch getrennt werden. Für die Anwendung in Speicherschichten werden jedoch üblicherweise die Isomerengemische eingesetzt.

Das Herstellverfahren ist an sich bekannt und beispielsweise in Angew. Chem. 78, 937 (1966), oder in der älteren deutschen Patentanmeldung DE-A-3 733 173 beschrieben.

Die Erfindung betrifft weiterhin neue Urethangruppen aufweisende Azulene der Formel II

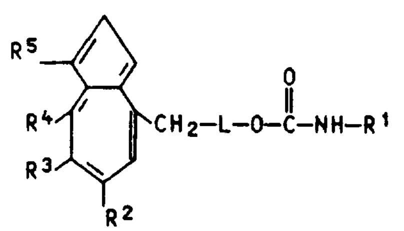

(II),

in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten. |

Bezüglich der beispielhaften Veranschaulichung der Reste L, $R^1$, $R^2$, $R^3$, $R^4$ und $R^5$ sei auf die voranstehend vorgenommene Aufzählung verwiesen.

Die Urethangruppen aufweisenden Azulenderivate der Formel II erhält man beispielsweise, wenn man von den entsprechenden Hydroxyalkylazulenderivaten ausgeht. Die Herstellung dieser Produkte ist beispielsweise in der älteren deutschen Patentanmeldung DE-A-3 733 173 beschrieben.

So eignen sich z.B. diejenigen Azulenderivate der Formel IVa oder IVb

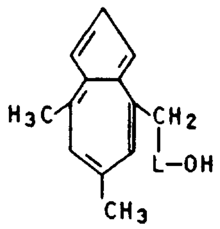

(IVa)

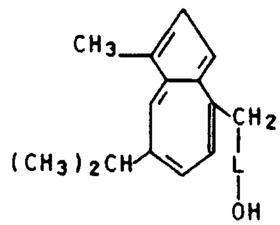

(IVb)

wobei L jeweils die obengenannte Bedeutung besitzt, besonders zur Umsetzung.

Als Reaktionspartner für die Hydroxyalkylazulenderivate dienen organische Monoisocyanate der Formel V

$R^1\text{-}N=C=O$ (V),

in der $R^1$ die obengenannte Bedeutung besitzt.

Die Umsetzung des Hydroxyalkylazulenderivats mit dem organischen Monoisocyanat erfolgt nach an sich bekannten Methoden. Dazu wird z.B. das Hydroxyalkylazulen mit dem organischen Monoisocyanat in einem inerten organischen Lösungsmittel (z.B. Methylenchlorid, 1,1,2-Trichlorethan, Toluol, Ligroin oder Cyclohexan) bei einer Temperatur von 20 bis 60 °C, gegebenenfalls in Gegenwart eines Katalysators (z.B. tertiäre Amine, Tetraalkylammoniumhydroxide oder organische Zinnverbindungen) umgesetzt. Im allgemeinen wird das Isocyanat im Überschuß eingesetzt.

Eine weitere Aufgabe der vorliegenden Erfindung war es, ein neues optisches Aufzeichnungsmedium mit Azulenquadratsäurederivaten als Speichermaterialien bereitzustellen, das in einfacher Weise hergestellt werden kann, das gut beschreibbar und anschließend auch gut lesbar ist, wobei das Signal:Rausch-Verhältnis möglichst hoch sein sollte, und das eine hohe Stabilität der Speicherschichten aufweist.

Die Erfindung betrifft nun weiterhin ein optisches Aufzeichnungsmedium, enthaltend einen Träger sowie einen strahlungsempfindlichen, Farbstoff und gegebenenfalls Bindemittel enthaltende dünnen Beschichtungsfilm, wobei der Farbstoff die Formel I

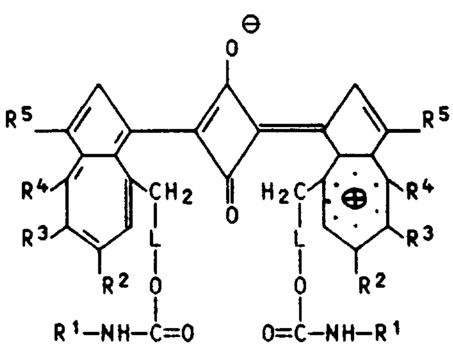

(I)

aufweist, in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten, |

mit der Maßgabe, daß wenn $R^5$ Wasserstoff bedeutet, an einem oder beiden Azulenringen die Ringpositionen der Substituenten $CH_2$-L-O-CO-$NHR^1$ und $R^4$ innerhalb eines Azulenrings auch gegenseitig vertauscht sein können.

Bevorzugt ist ein optisches Aufzeichnungsmedium, das Azulenquadratsäurefarbstoffe der Formel I enthält, in der $R^2$, $R^3$, $R^4$ und $R^5$ jeweils $C_1$-$C_6$-Alkyl bedeuten.

Weiterhin bevorzugt ist ein optisches Aufzeichnungsmedium, das Azulenquadratsäurefarbstoffe der Formel I enthält, in der $R^1$ $C_1$-$C_{18}$-Alkyl oder Cyclohexyl bedeutet.

Besonders bevorzugt ist ein optisches Aufzeichnungsmedium, das Azulenquadratsäurefarbstoffe der Formel I enthält, in der $R^2$ und $R^4$ jeweils Methyl und $R^3$ und $R^5$ jeweils Wasserstoff bedeuten.

Ganz besonders bevorzugt ist ein optisches Aufzeichnungsmedium, das Azulenquadratsäurefarbstoffe der Formel I enthält, in der $R^2$ und $R^4$ jeweils Wasserstoff, $R^3$ Isopropyl und $R^5$ Methyl bedeuten.

Als Träger kommen zweckmäßig transparente Träger, wie Glas oder Kunststoffe in Betracht. Geeignete Kunststoffe sind beispielsweise Poly(meth)-acrylate, Polycarbonate, Polyester, Epoxide, Polyolefine (z.B. Polymethylpenten), Polyamid, Polyvinylchlorid, Polystyrol oder Polyvinylester.

Ein bevorzugtes Aufzeichungsmedium weist einen Träger aus Polycarbonat oder Poly(meth)acrylaten, insbesondere aber aus Polycarbonat auf.

Weiterhin bevorzugt ist ein optisches Aufzeichnungsmedium, das 1 bis 30 Gew.-%, bezogen auf den Farbstoff, an Bindemittel enthält.

Die neuen Azulenquadratsäurefarbstoffe der Formel I zeigen gute optische Daten. Darüberhinaus sind bei den neuen Verbindungen die reinen Farbstoffschichten sehr stabil. Bislang wurde nämlich keine Rekristallisation der reinen Farbstoffschicht beobachtet und somit kann auf den Zusatz polymerer Bindemittel verzichtet werden. Außerdem ist auch die Lichtechtheit (Stabilität) deutlich größer als bei bekannten Methinfarbstoffen, so daß der Zusatz von Stabilisatoren zur Schichtrezeptur auf ein Minimum begrenzt werden kann. Von besonderem Vorteil ist auch die gute Löslichkeit der neuen Farbstoffe I in den meisten organischen Lösungsmitteln, so daß diese Farbstoffe direkt (ohne Schutzschicht) auf strukturierte Kunststoffsubstrate, insbesondere Polycarbonatsubstrate, aufgeschleudert werden können.

Wie oben ausgeführt, enthält die aufzuschleudernde Lösung vorzugsweise ein Bindemittel, um eine gute Langzeitstabilität des Aufzeichnungsmediums zu gewährleisten und vor allem die optimale Viskosität der aufzuschleudernden Lösung einstellen zu können. Vorzugsweise enthält die Lösung dabei 1 bis 30 Gew.%, bezogen auf den Gehalt an gelöstem Feststoff in der aufzuschleudernden Lösung, eines Bindemittels. Als Bindemittel kommen z.B. Polyorganosiloxane, Epoxide, Poly(meth)acrylate, Polystyrolhomo- und -copolymerisate, Polyvinylcarbazol, Polyvinylpyrrolidon, Polyimidazolcopolymere, Polyvinylestercopolymere, Polyvinylethercopolymere, Polyvinylidenchloridcopolymere, Acrylnitrilcopolymere, Polyvinylchlorid oder dessen Copolymere, Celluloseacetat oder Nitrocellulose in Betracht.

Ein bevorzugtes Aufzeichungsmedium weist ein Bindemittel auf Basis eines Vinylpyrrolidon-Vinylacetat-Copolymeren oder eines Polyvinylchlorid-Polyvinylether-Copolymeren auf.

Das erfindungsgemäße optische Aufzeichungsmedium wird zweckmäßig durch Aufschleudern einer Lösung, enthaltend organisches Lösungsmittel, Azulenquadratsäurefarbstoff I und gegebenenfalls Bindemittel hergestellt. Zweckmäßig weist die aufzuschleudernde Lösung dabei einen Gehalt an gelöstem Feststoff von 1 bis 30 Gew.%, bezogen auf die Lösung, auf.

Geeignete Lösungsmittel sind z.B. Propanol, Isopropanol, Butanol Diacetonalkohol, Methylethylketon, Toluol, Bromoform, 1,1,2-Trichlorethan oder deren Mischungen.

Gegebenenfalls kann die Lösung noch bis zu 10 Gew.-%, bezogen auf den Gehalt an gelöstem Feststoff in der aufzuschleudernden Lösung, an Additiven, z.B. Antioxidantien, Singlett-Sauerstoff-Quencher oder UV-Absorber enthalten.

Bevorzugt enthält die aufzuschleudernde Lösung bis zu 5 Gew.-%, bezogen auf den Gehalt an gelöstem Feststoff in der aufzuschleudernden Lösung, einer Mischung von mehreren Antioxidantien, Singlett-Sauerstoff-Quenchern und UV Absorbern. Bei Anwendung von solchen Antioxidantien, die ebenfalls im nahen Infrarot absorbieren, beispielsweise Nickeldithiolenkomplexe, wie sie z.B. in der DE-A 3 505 750, DE-A 3 505 751 oder in S.H. Kim, M. Matsuoka, M. Yomoto, Y. Tsuchiya u. T. Kitao, Dyes and Pigments, Bd. 8, S. 381-388 (1987), beschrieben sind können vorzugsweise bis zu 10 Gew.-%, bezogen auf den Gehalt an gelöstem Feststoff in der aufzuschleudernden Lösung, in der Lösung enthalten sein.

Unter Aufschleudern ist dabei das Aufbringen der Lösung auf den in Rotation befindlichen Träger, der zweckmäßig eine runde Form aufweist, zu verstehen. Es ist aber auch möglich, die Lösung auf den zunächst ruhenden Träger aufzubringen und ihn anschließend in Rotation zu versetzen. Das Aufgeben der Lösung auf den Träger erfolgt zweckmäßig mit einer Spritze oder Kapillaren oder mittels einer mechanischen Pumpe.

Die Rotation des Trägers erfolgt im allgemeinen mit einer Geschwindigkeit von 50 bis 7000 U/min, vorzugsweise 500 bis 5000 U/min, wobei das Aufschleudern der Lösung zweckmäßig bei geringerer Drehzahl (ca. 500 bis 2000 U/min) und das daran anschließende Trockenschleudern bei höherer Drehzahl (ca. 5000 bis 7000 U/min) vorgenommen wird. Die Schichtdicke der gegenüber Laserlicht empfindlichen Schicht beträgt 40 bis 160 nm, vorzugsweise 80 bis 120 nm. Sie ist abhängig von der Drehzahl, von der Konzentration und Viskosität der aufzuschleudernden Lösung sowie von der Temperatur.

Beim erfindungsgemäßen optischen Aufzeichnungsmedium liegt die gegenüber Laserlicht empfindliche Schicht in Form einer homogenen, dünnen, glatten Schicht vor, die eine hohe optische Qualität aufweist. So liegen die Reflektivitätswerte im allgemeinen in einem Bereich der größer als 12 % ist.

Das neue Aufzeichungsmedium ist ferner bei der Wellenlänge der verwendeten Laserlichtquelle hinreichend empfindlich, d. h. bei Einstrahlung von Lichtpulsen von wenigen nJ Energieinhalt, die auf Brennpunktdurchmesser von ≦ 1 µm fokussiert sind, bilden sich Pits aus, wobei ein ausgezeichnetes Signal: Rausch-Verhalten erzielt wird.

Als Laserlichtquelle eignen sich wegen der geringen Größe des Bauelementes, seines geringen Energiebedarfs und der Möglichkeit der direkten Modulation der optischen Ausgangsleistung durch Modulation des elektrischen Antriebstromes Festkörper-Injektionslaser, die im nahen Infrarot emittieren, vor allem der AlGaAs-Laser, der im Wellenlängenbereich zwischen etwa 700 und 900 nm arbeitet, besonders gut.

Die folgenden Beispiele sollen die Erfindung näher erläutern.

Beispiel 1

Herstellung von N-(n-Butyl)-0-propyl-3-[7-isopropyl-1-methylazulen-4-yl]urethan, (Verb. Nr. 2)

Zu einer Lösung von 9,6 g (0,04 mol) 7-Isopropyl-1-methyl-4-(3-hydroxypropyl)azulen in 100 ml Methylenchlorid wurden bei Raumtemperatur 150 g (0,15 mol) n-Butylisocyanat zugetropft und anschließend die Reaktionslösung 5 Stunden unter Rückfluß erhitzt. Danach wurde das Lösungsmittel abdestilliert und der Rückstand über Kieselgel chromatographiert (Methylenchlorid/Aceton). Man erhielt 7,0 g (51 %) des Urethans als blaues, hochviskoses Öl (Verb. Nr. 2).

Physikalische Daten:

IR (KBr) : $\bar{\nu}$ = 3336 (breit, NH), 2958, 2931, 2871, 1699 s (C=0), 1527, 1465, 1387, 1251, 1142, 1061, 1030, 784 $cm^{-1}$; 1H-NMR ($CDCl_3$) : $\delta$ = 0,90 t(3H) , 1.25 -1.55 m, 1.38 d(6H) , 2.17 q(2H) , 2.66 s(3H), 3.08 q(1H), 3.17 m, 4,5 t(2H) , 4.46 breit (NH), 6.98 d(1H), 7.28 d(1H), 7.39 d( 1H), 7.61 d(1H), 8.18 s(1H); $^{13}C$-NMR ($CDCl_3$) : $\delta$ = 12.86, 13.69, 20.00, 24.75 (2C), 30.79, 32.34, 34.65, 38.34, 41.04, 64.71, 112.40, 124.51, 125.44, 133.26, 135.15, 136.48, 136.59, 137.41, 140.02, 147.92, 156.82;

MS:m/e = 341 (100 %), $M^{\oplus}$), 326, 312, 298, 258, 240, 224 (45 %), 209, 198 (60 %), 181.

Analog Beispiel 1 wurden die in Tabelle 1 aufgeführten Azulenderivate hergestellt. Ihre Struktur ist durch IR-, 1H-NMR-, $^{13}$-C-NMR- und MS-Spektren abgesichert.

Tabelle 1

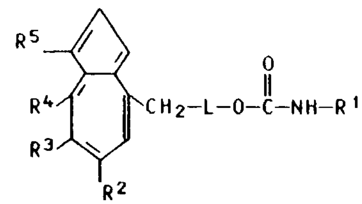

| Verb.-Nr. | $L-O-C(O)NH-R^1$ | $R^2$ | $R^3$ | $R^4$ | $R^5$ | MS $[M^{\oplus}/100\%]$ | IR ($\bar{\nu}/cm^{-1}$) [NH, C=O] |
|---|---|---|---|---|---|---|---|
| 1 | $(CH_2)_2OC(O)NH-C_6H_5$ | H | $CH(CH_3)_2$ | H | $CH_3$ | 361 | 3340, 1699 |
| 2 | $(CH_2)_2OC(O)NH-nC_4H_9$ | H | $CH(CH_3)_2$ | H | $CH_3$ | 341 | 3340, 1695 |
| 3 | $(CH_2)_2OC(O)NH-tC_4H_9$ | H | $CH(CH_3)_2$ | H | $CH_3$ | 341 | 3340, 1696 |
| 4 | $(CH_2)_2OC(O)NH-iC_4H_9$ | H | $CH(CH_3)_2$ | H | $CH_3$ | 327 | 3340, 1695 |
| 5 | $(CH_2)_2OC(O)NH-$ 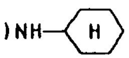 | H | $CH(CH_3)_2$ | H | $CH_3$ | 367 | 3340, 1697 |
| 6 | $(CH_2)_2OC(O)NH-nC_{18}H_{37}$ | H | $CH(CH_3)_2$ | H | $CH_3$ | 537 | 3340, 1693 Fp.:37-38°C |
| 7 | $(CH_2)_3OC(O)NH-C_6H_5$ | H | $CH(CH_3)_2$ | H | $CH_3$ | 375 | 3340, 1696 |
| 8 | $(CH_2)_3OC(O)NH-nC_4H_9$ | H | $CH(CH_3)_2$ | H | $CH_3$ | 355 | 3340, 1697 |
| 9 | $(CH_2)_3OC(O)NH-tC_4H_9$ | H | $CH(CH_3)_2$ | H | $CH_3$ | 355 | 3340, 1695 |
| 10 | $(CH_2)_3OC(O)NH-iC_3H_7$ | H | $CH(CH_3)_2$ | H | $CH_3$ | 341 | 3340, 1695 |
| 11 | $(CH_2)_3OC(O)NH-$ 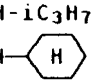 | H | $CH(CH_3)_2$ | H | $CH_3$ | 381 | 3340, 1696 |
| 12 | $(CH_2)_3OC(O)NH-nC_{18}H_{37}$ | H | $CH(CH_3)_2$ | H | $CH_3$ | 551 | 3340, 1694 |
| 13 | $CHCH_2OC(O)NH-C_6H_5$<br>\|<br>$CH_3$ | H | $CH(CH_3)_2$ | H | $CH_3$ | 375 | 3340, 1698 |
| 14 | $CHCH_2OC(O)NH-nC_4H_9$<br>\|<br>$CH_3$ | H | $CH(CH_3)_2$ | H | $CH_3$ | 355 | 3340, 1698 |

Tabelle 1 (Fortsetzung)

| Verb.-Nr. | L–O–C(O)NH–$R^1$ | $R^2$ | $R^3$ | $R^4$ | $R^5$ | MS [$M^{\oplus}$/100%] | IR ($\bar{\nu}$/$cm^{-1}$) [NH, C=O] |
|---|---|---|---|---|---|---|---|
| 15 | $CHCH_2OC(O)NH$-$tC_4H_9$<br>\|<br>$CH_3$ | H | $CH(CH_3)_2$ | H | $CH_3$ | 355 | 3340, 1696 |
| 16 | $CHCH_2OC(O)NH$-$iC_3H_7$<br>\|<br>$CH_3$ | H | $CH(CH_3)_2$ | H | $CH_3$ | 341 | 3340, 1697 |
| 17 | $CHCH_2OC(O)NH$– 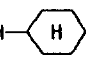<br>\|<br>$CH_3$ | H | $CH(CH_3)_2$ | H | $CH_3$ | 381 | 3340, 1696 |
| 18 | $CHCH_2OC(O)NH$-$nC_{18}H_{37}$<br>\|<br>$CH_3$ | H | $CH(CH_3)_2$ | H | $CH_3$ | 551 | 3340, 1695 |
| 19 | $CHCH_2OC(O)NH$-$nC_4H_9$<br>\|<br>$C_2H_5$ | H | $CH(CH_3)_2$ | H | $CH_3$ | 369 (70%)<br>198(100%) | 3340, 1698 |
| 20 | $CHCH_2OC(O)NH$-$tC_4H_9$<br>\|<br>$C_2H_5$ | H | $CH(CH_3)_2$ | H | $CH_3$ | 369 | 3340, 1696 |
| 21 | $CHCH_2OC(O)NH$-iC–$H_7$<br>\|<br>$C_2H_5$ | H | $CH(CH_3)_2$ | H | $CH_3$ | 355 | 3340, 1697 |
| 22 | CH-$CH_2OC(O)NH$– 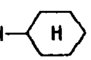<br>\|<br>$C_2H_5$ | H | $CH(CH_3)_2$ | H | $CH_3$ | 395 | 3340, 1696 |
| 23 | CH-$CH_2OC(O)NH$-$nC_{18}H_{37}$<br>\|<br>$C_2H_5$ | H | $CH(CH_3)_2$ | H | $CH_3$ | 565 | 3340, 1695 |
| 24 | CH-$CH_2OC(O)NH$-$nC_4H_9$<br>\|<br>$C_6H_5$ | H | $CH(CH_3)_2$ | H | $CH_3$ | 417 (40%)<br>198(100%) | 3340, 1696 |
| 25 | $(CH_2)_2OC(O)NH$-$nC_4H_9$ | $CH_3$ | H | $CH_3$ | H | | 3350, 1690 |

Beispiel 2

Darstellung des Bis (0-propyl-3-[7-isopropyl-1-methyl-azulen-4-yl]-N-(n-butyl)urethan)quadratsäurefarbstoffs (Verb. Nr. 27).

6,8 g (0,002 mol) N-(n-Butyl)-0-propyl-3-[7-isopropyl-1-methylazulen-4-yl]-urethan (Beispiel 1) und 2,3 g (0,02 mol) Quadratsäure wurden in 160 ml Toluol/n-Butanol (1:1) 5 Stunden unter Rückfluß erhitzt. Das nach Abziehen des Lösungsmittels unter vermindertem Druck verbleibende grüne Öl wurde über Kieselgel chromatographiert (Methylenchlorid/Aceton).

Ausbeute: 2,3 g (30 %) metallisch glänzende Kristalle;
Physikalische Daten:
Schmp. : 155-156 °C
UV ($CH_2Cl_2$): λmax = 770 nm (ε = 112 250); IR (KBR) : $\bar{\nu}$ = 3325 (NH), 2958, 2928, 2862, 1691, 1609, 1592, 1543, 1432, 1387, 1326s, 1248, 1217, 1024, 916, 765, 587 $cm^{-1}$;
$^1H$-NMR ($CDCl_3$): δ = 0.84 t(6H), 1.13-1.45 m(8H), 1.48 d(12H), 1.91 q(4H), 2.55 s(6H), 2.98 q(4H), 3.11 q-(2H), 3.88 m(4H), 3.98 m(4H), 4.74 breit (2NH), 7.45 d(2H), 7.58 d(2H), 8.09 s(2H), 8.82 s(2H);
$^{13}C$-NMR ($CDCl_3$): δ = 12.96 (2C), 13.66 (2C), 19.95 (2C), 24.24 (4C), 31.02 (2C), 32.16 (2C), 36.30 (2C), 38.40 (2C), 40.91 (2C), 63.97 (2C), 121.66 (2C), 130.82 (2C), 133.98 (2C), 134.55 (2C), 138.24 (2C), 139.85 (2C), 141.83 (2C), 147.50 (2C), 150.27 (2C), 156.01 (2C), 156.72 (2C), 179.53, 182.75, 153. 24 (2C);
MS:m/e = 762, 760 (40 %)
Analog Beispiel 2 wurden die in Tabelle 2 aufgeführten Quadratsäurefarbstoffe hergestellt und zusätzlich durch $^1H$-NMR-, $^{13}C$-NMR-, IR- und MS-Spektren charakterisiert.

Tabelle 2

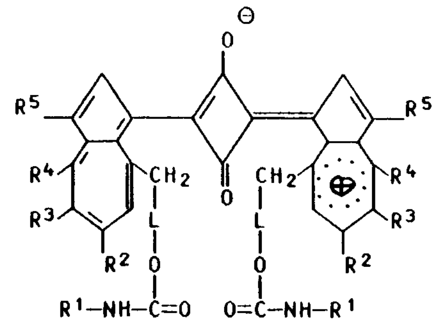

| Verb.-Nr. | L-O-C(O)NH-$R^1$ | $R^2$ | $R^3$ | $R^4$ | $R^5$ | λ max [nm] (in $CH_2Cl_2$) | Schmp. [°C] |
|---|---|---|---|---|---|---|---|
| 26 | $(CH_2)_2OC(O)NH-C_6H_5$ | H | $CH(CH_3)_2$ | H | $CH_3$ | 770 | 257-258 |
| 27 | $(CH_2)_2OC(O)NH-nC_4H_9$ | H | $CH(CH_3)_2$ | H | $CH_3$ | 770 | 155-156 vgl. Beisp. 2 |
| 28 | $(CH_2)_2OC(O)NH-tC_4H_9$ | H | $CH(CH_3)_2$ | H | $CH_3$ | 768 | 190-192 |
| 29 | $(CH_2)_2OC(O)NH-iC_3H_7$ | H | $CH(CH_3)_2$ | H | $CH_3$ | 768 | 143-144 |
| 30 | $(CH_2)_2OC(O)NH-$ 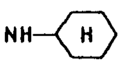 | H | $CH(CH_3)_2$ | H | $CH_3$ | 768 | 197 |
| 31 | $(CH_2)_2OC(O)NH-nC_{18}H_{37}$ | H | $CH(CH_3)_2$ | H | $CH_3$ | 770 | 100-101 |
| 32 | $(CH_2)_3OC(O)NH-C_6H_5$ | H | $CH(CH_3)_2$ | H | $CH_3$ | 769 | 238-242 |
| 33 | $(CH_2)_3OC(O)NH-nC_4H_9$ | H | $CH(CH_3)_2$ | H | $CH_3$ | 768 | 133-134 |
| 34 | $(CH_2)_3OC(O)NH-tC_4H_9$ | H | $CH(CH_3)_2$ | H | $CH_3$ | 768 | 157-158 |
| 35 | $(CH_2)_3OC(O)NH-iC_3H_7$ | H | $CH(CH_3)_2$ | H | $CH_3$ | 768 | 154-155 |
| 36 | $(CH_2)_3OC(O)NH-$ 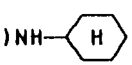 | H | $CH(CH_3)_2$ | H | $CH_3$ | 768 | 185-186 |
| 37 | $(CH_2)_3OC(O)NH-nC_{18}H_{37}$ | H | $CH(CH_3)_2$ | H | $CH_3$ | 769 | 97- 98 |

Tabelle 2 (Fortsetzung)

| Verb.-Nr. | $L-O-C(O)NH-R^1$ | $R^2$ | $R^3$ | $R^4$ | $R^5$ | λ max [nm] (in $CH_2Cl_2$) | Schmp. [°C] |
|---|---|---|---|---|---|---|---|
| 38 | $CHCH_2OC(O)NH-C_6H_5$<br>\|<br>$CH_3$ | H | $CH(CH_3)_2$ | H | $CH_3$ | 772 | 208-212 |
| 39 | $CHCH_2OC(O)NH-nC_4H_9$<br>\|<br>$CH_3$ | H | $CH(CH_3)_2$ | H | $CH_3$ | 773 | 185-187* |
| 40 | $CHCH_2OC(O)NH-tC_4H_9$<br>\|<br>$CH_3$ | H | $CH(CH_3)_2$ | H | $CH_3$ | 773 | 174-175* |
| 41 | $CHCH_2OC(O)NH-iC_3H_7$<br>\|<br>$CH_3$ | H | $CH(CH_3)_2$ | H | $CH_3$ | 773 | 168-169* |
| 42 | $CHCH_2OC(O)NH-$ 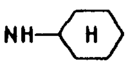<br>\|<br>$CH_3$ | H | $CH(CH_3)_2$ | H | $CH_3$ | 773 | 197-198* |
| 43 | $CHCH_2OC(O)NH-nC_{18}H_{37}$<br>\|<br>$CH_3$ | H | $CH(CH_3)_2$ | H | $CH_3$ | 773 | 93- 74* |
| 44 | $CHCH_2OC(O)NH-nC_4H_9$<br>\|<br>$C_2H_5$ | H | $CH(CH_3)_2$ | H | $CH_3$ | 772 | 209* |
| 45 | $CHCH_2OC(O)NH-tC_4H_9$<br>\|<br>$C_2H_5$ | H | $CH(CH_3)_2$ | H | $CH_3$ | 772 | 201-202* |
| 46 | $CHCH_2OC(O)NH-iC_3H_7$<br>\|<br>$C_2H_5$ | H | $CH(CH_3)_2$ | H | $CH_3$ | 770 | 90-115* |

Tabelle 2 (Fortsetzung)

| Verb.-Nr. | L-O-C(O)NH-$R^1$ | $R^2$ | $R^3$ | $R^4$ | $R^5$ | λ max [nm] (in $CH_2Cl_2$) | Schmp. [°C] |
|---|---|---|---|---|---|---|---|
| 47 | $CHCH_2OC(O)NH$– 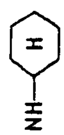<br>\|<br>$C_2H_5$ | H | $CH(CH_3)_2$ | H | $CH_3$ | 770 | 60- 85* |
| 48 | $CHCH_2OC(O)NH$-$nC_{18}H_{37}$<br>\|<br>$C_2H_5$ | H | $CH(CH_3)_2$ | H | $CH_3$ | 774 | [-Öl-]* |
| 49 | $CHCH_2OC(O)NH$-$nC_4H_9$<br>\|<br>$C_6H_5$ | H | $CH(CH_3)_2$ | H | $CH_3$ | 774 | 102* |
| 50 | $(CH_2)_2OC(O)NH$-$C_4H_9$ | $CH_3$ | H | $CH_3$ | H | 720 | |

*) Das Produkt ist ein Isomerengemisch oder nicht analysenrein.

Beispiel 3

Eine 5 gew.%ige Lösung des Farbstoffs 27 in Toluol wurde mit einer Spritze bei ca. 2000 U/min auf eine rotierende Polymethylmethacrylatscheibe aufgetragen und dann das restliche Lösungsmittel bei 5000 U/min abgeschleudert, Man erhielt eine homogene, hochreflektierende Farbstoffschicht, welche sich mit einem Halbleiterlaser (λ = 830 nm) sehr gut beschreiben ließ. Die Informationen können mit gutem

Kontrast wieder ausgelesen werden.

Beispiel 4

Eine 3 gew.%ige Lösung des Farbstoffs 27, welche 30 Gew.%, bezogen auf den Gehalt an gelöstem Feststoff in der Lösung, Polymethylmethacrylat enthielt, wurde analog Beispiel 3 auf eine gerillte Polycarbonatscheibe aufgeschleudert. Man erhielt eine homogene, hochreflektierende Farbstoffschicht, die auf dem Substrat gut haftet, die Spurrillen des Substrats gut abbildet und mit einem Halbleiterlaser ($\lambda$ = 830 nm) sehr gut beschreibbar war. Die eingeschriebene Information war im Klimatest stabil und kann mit gutem Kontrast beliebig oft wieder ausgelesen werden.

Beispiel 5

Eine 2 gew.%ige Lösung des Farbstoffs 27 in Propanol/Diacetonalkohol 1:1, welche, bezogen auf den Gehalt an gelöstem Feststoff in der Lösung, 10 Gew.% eines Phenolharzes als Bindemittel und 5 Gew.% 4-Octyl-4'-fluordiphenyldithiolennickel als Stabilisator enthielt, wurde analog Beispiel 3 auf eine gerillte Polycarbonatscheibe aufgeschleudert. Die erhaltene Speicherschicht war derjenigen aus Beispiel 3 in allen Belangen vergleichbar, wies aber eine erhöhte Stabilität gegenüber UV-Licht auf.

Beispiel 6

Eine 2 gew.%ige Lösung des Farbstoffs 48 in Toluol, welche, bezogen auf den Gehalt an gelöstem Feststoff in der Lösung, 10 Gew.% Polymethylmethacrylat und 5 Gew.% Biscampheratodithiolennickel enthielt, wurde analog Beispiel 3 auf eine Glasscheibe aufgeschleudert. Die erhaltene Farbstoffschicht war homogen und zeigte eine hohe Grundreflektivität. Sie konnte mit einem Halbleiterlaser ($\lambda$ = 780 nm) gut beschrieben werden, Die eingeschriebenen Informationen sind unter den üblichen Testbedingungen stabil und können beliebig oft wieder ausgelesen werden.

**Patentansprüche**

**1.** Urethangruppen aufweisende Azulenquadratsäurefarbstoffe der Formel I

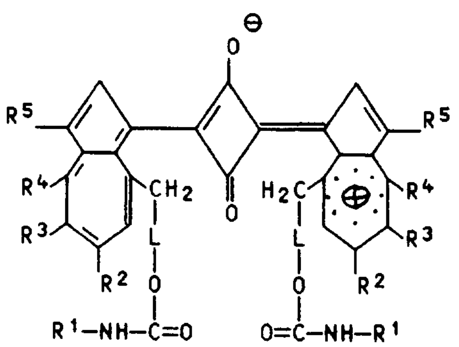

(I),

in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten, |

mit der Maßgabe, daß wenn $R^5$ Wasserstoff bedeutet, an einem oder beiden Azulenringen die Ringpositionen der Substituenten $CH_2$-L-O-CO-NH$R^1$ und $R^4$ innerhalb eines Azulenrings auch gegeneinander vertauscht sein können.

**2.** Azulenquadratsäurefarbstoffe gemäß Anspruch 1, dadurch gekennzeichnet, daß $R^2$, $R^3$, $R^4$ und $R^5$ jeweils $C_1$-$C_6$-Alkyl bedeuten.

3. Azulenquadratsäurefarbstoffe gemäß Anspruch 1, dadurch gekennzeichnet, daß $R^2$ und $R^4$ jeweils Methyl und $R^3$ und $R^5$ jeweils Wasserstoff bedeuten.

4. Azulenquadratsäurefarbstoffe gemäß Anspruch 1, dadurch gekennzeichnet, daß $R^2$ und $R^4$ jeweils Wasserstoff, $R^3$ Isopropyl und $R^5$ Methyl bedeuten.

5. Optisches Aufzeichnungsmedium, enthaltend einen Träger sowie einen strahlungsempfindlichen, Farbstoff und gegebenenfalls Bindemittel enthaltenden dünnen Beschichtungsfilm, dadurch gekennzeichnet, daß der Farbstoff die Formel I

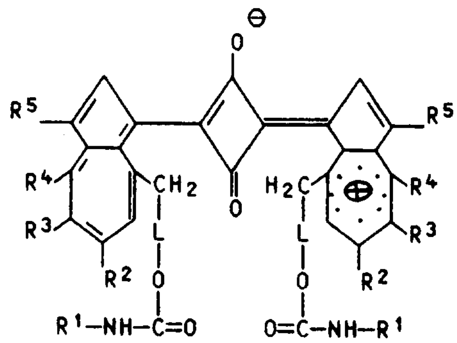

(I),

aufweist, in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten, |

mit der Maßgabe, daß wenn $R^5$ Wasserstoff bedeutet, an einem oder beiden Azulenringen die Ringpositionen der Substituenten $CH_2$-L-O-CO-$NHR^1$ und $R^4$ innerhalb eines Azulenringes auch gegeneinander vertauscht sein können.

6. Optisches Aufzeichnungsmedium gemäß Anspruch 1, dadurch gekennzeichnet, daß $R^2$, $R^3$, $R^4$ und $R^5$ jeweils $C_1$-$C_6$-Alkyl bedeuten.

7. Optisches Aufzeichnungsmedium gemäß Anspruch 1, dadurch gekennzeichnet, daß $R^2$ und $R^4$ jeweils Methyl und $R^3$ und $R^5$ jeweils Wasserstoff bedeuten.

8. Optisches Aufzeichnungsmedium gemäß Anspruch 1, dadurch gekennzeichnet, daß $R^2$ und $R^4$ jeweils Wasserstoff, $R^3$ Isopropyl und $R^5$ Methyl bedeuten.

9. Urethangruppen aufweisende Azulene der Formel II

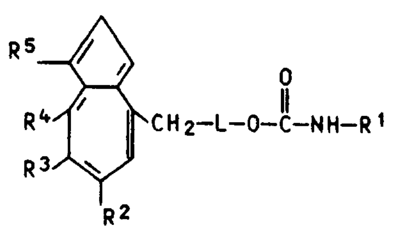

(II),

in der

| | |
|---|---|
| L | $C_1$-$C_{12}$-Alkylen, das gegebenenfalls durch Phenyl substituiert ist, |
| $R^1$ | $C_1$-$C_{20}$-Alkyl, $C_5$-$C_7$-Cycloalkyl oder gegebenenfalls substituiertes Phenyl und |

$R^2$, $R^3$, $R^4$ und $R^5$ gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, bedeuten.

## Claims

**1.** An azulenesquaric acid dye that contains urethane groups and has the formula I

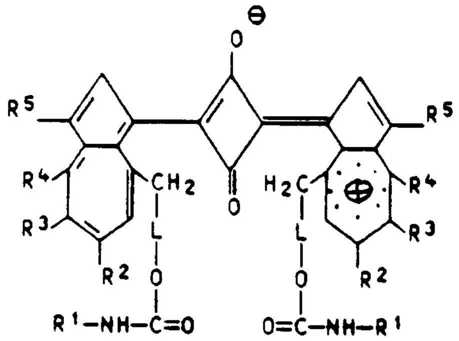

(I),

where

- L is $C_1$-$C_{12}$-alkylene, which may be substituted by phenyl,
- $R^1$ is $C_1$-$C_{20}$-alkyl, $C_5$-$C_7$-cycloalkyl or substituted or unsubstituted phenyl and
- $R^2$, $R^3$, $R^4$ and $R^5$ are identical or different and are each independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or cyano,

with the proviso that when $R^5$ is hydrogen the positions of the substituents $CH_2$-L-O-CO-$NHR^1$ and $R^4$ on an azulene ring may also be interchanged within a ring in either or both of the azulene rings.

**2.** An azulenesquaric acid dye as claimed in claim 1, wherein $R^2$, $R^3$, $R^4$ and $R^5$ are each $C_1$-$C_6$-alkyl.

**3.** An azulenesquaric acid dye as claimed in claim 1, wherein $R^2$ and $R^4$ are each methyl and $R^3$ and $R^5$ are each hydrogen.

**4.** An azulenesquaric acid dye as claimed in claim 1, wherein $R^2$ and $R^4$ are each hydrogen, $R^3$ is isopropyl and $R^5$ is methyl.

**5.** An optical recording medium comprising a base and a thin radiation sensitive coating film containing a dye with or without a binder, wherein the dye has the formula I

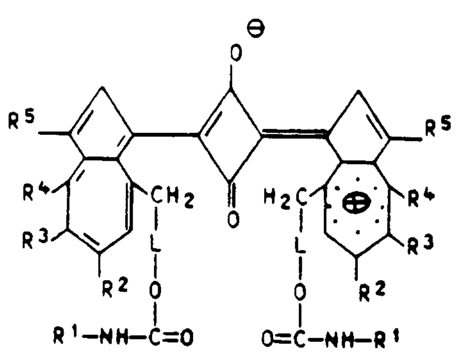

(I),

where

- L is $C_1$-$C_{12}$-alkylene, which may be substituted by phenyl,

| | |
|---|---|
| $R^1$ | is $C_1$-$C_{20}$-alkyl, $C_5$-$C_7$-cycloalkyl or substituted or unsubstituted phenyl and |
| $R^2$, $R^3$, $R^4$ and $R^5$ | are identical or different and are each independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or cyano, |

with the proviso that when $R^5$ is hydrogen the positions of the substituents $CH_2$-L-O-CO-NHR$^1$ and $R^4$ on an azulene ring may also be interchanged within a ring in either or both of the azulene rings.

6. An optical recording medium as claimed in claim 5, wherein $R^2$, $R^3$, $R^4$ and $R^5$ are each $C_1$-$C_6$-alkyl.

7. An optical recording medium as claimed in claim 5, wherein $R^2$ and $R^4$ are each methyl and $R^3$ and $R^5$ are each hydrogen.

8. An optical recording medium as claimed in claim 5, wherein $R^2$ and $R^4$ are each hydrogen, $R^3$ is isopropyl and $R^5$ is methyl.

9. An azulene that contains urethane groups and has the formula II

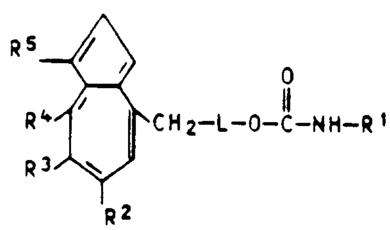

(II),

where

| | |
|---|---|
| L | is $C_1$-$C_{12}$-alkylene, which may be substituted by phenyl, |
| $R^1$ | is $C_1$-$C_{20}$-alkyl, $C_5$-$C_7$-cycloalkyl or substituted or unsubstituted phenyl and |
| $R^2$, $R^3$, $R^4$ and $R^5$ | are identical or different and are each independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or cyano. |

## Revendications

1. Colorants du type acide azulènequadratique présentant des radicaux uréthanne de la formule I

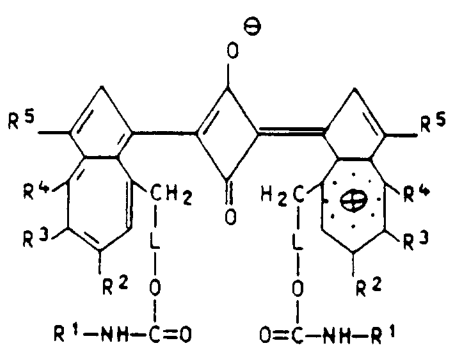

(I)

dans laquelle

| | |
|---|---|
| L | représente un radical alkylène à substitution phénylique éventuelle, |
| $R^1$ | représente un radical alkyle en $C_1$ à $C_{20}$, cycloalkyle en $C_5$ à $C_7$, ou phényle éventuellement substitué et |
| $R^2$, $R^3$, $R^4$ et $R^5$ | sont identiques ou différents et représentent chacun indépendamment des autres, un atome d'hydrogène ou un radical alkyle en $C_1$ à $C_{12}$ qui est éventuellement substitué par des halogènes, des radicaux alcoxy en $C_1$ à $C_{12}$, |

phényle, phényle substitué, alcoxy($C_1$-$C_{12}$)carbonyle, ou cyano,

avec la condition que lorsque $R^5$ représente un atome d'hydrogène, les positions cycliques sur l'un ou les deux cycles azulène des substituants $CH_2$-L-O-CO-NHR$^1$ et $R^4$ peuvent aussi être substituées l'une à l'autre à l'intérieur d'un cycle azulène.

2. Colorants du type acide azulènequadratique selon la revendication 1, caractérisés en ce que $R^2$, $R^3$, $R^4$ et $R^5$ représentent chacun un radical alkyle en $C_1$-$C_6$.

3. Colorants du type acide azulènequadratique selon la revendication 1, caractérisés en ce que $R^2$ et $R^4$ représentent chacun le radical méthyle et $R^3$ et $R^5$ représentent chacun un atome d'hydrogène.

4. Colorants du type acide azulènequadratique selon la revendication 1, caractérisés en ce que $R^2$ et $R^4$ représentent chacun un atome d'hydrogène, $R^3$ représente le radical isopropyle et $R^5$ représente le radical méthyle.

5. Milieu d'enregistrement optique contenant un support, comme aussi un colorant photosensible et un mince film de revêtement contenant éventuellement un liant, caractérisé en ce que le colorant répond à la formule I suivante

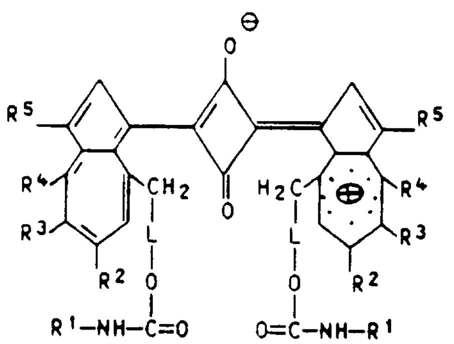

(I),

dans laquelle

| | |
|---|---|
| L | représente un radical alkylène à substitution phénylique éventuelle, |
| $R^1$ | représente un radical alkyle en $C_1$ à $C_{20}$, cycloalkyle en $C_5$ à $C_7$, ou phényle éventuellement substitué et |
| $R^2$, $R^3$, $R^4$ et $R^5$ | sont identiques ou différents et représentent chacun indépendamment des autres, un atome d'hydrogène ou un radical alkyle en $C_1$ à $C_{12}$ qui est éventuellement substitué par des halogènes, des radicaux alcoxy en $C_1$ à $C_{12}$, phényle, phényle substitué, alcoxy($C_1$-$C_{12}$)carbonyle, ou cyano, |

avec la condition que lorsque $R^5$ représente un atome d'hydrogène, les positions cycliques sur l'un ou les deux cycles azulène des substituants $CH_2$-L-O-CO-NHR$^1$ et $R^4$ peuvent aussi être substituées l'une à l'autre à l'intérieur d'un cycle azulène.

6. Milieu d'enregistrement optique suivant la revendication 1, caractérisés en ce que $R^2$, $R^3$, $R^4$ et $R^5$ représentent chacun un radical alkyle en $C_1$-$C_6$.

7. Milieu d'enregistrement optique suivant la revendication 1, caractérisés en ce que $R^2$ et $R^4$ représentent chacun le radical méthyle et $R^3$ et $R^5$ représentent chacun un atome d'hydrogène.

8. Milieu d'enregistrement optique suivant la revendication 1, caractérisés en ce que $R^2$ et $R^4$ représentent chacun un atome d'hydrogène, $R^3$ représente le radical isopropyle et $R^5$ représente le radical méthyle.

**9.** Azulènes présentant des radicaux uréthanne de la formule II

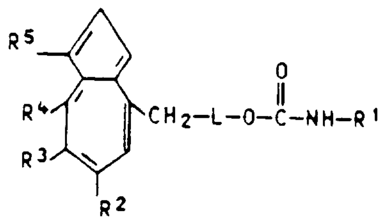

(II)

dans laquelle

| | |
|---|---|
| L | représente un radical alkylène à substitution phénylique éventuelle, |
| $R^1$ | représente un radical alkyle en $C_1$ à $C_{20}$, cycloalkyle en $C_5$ à $C_7$, ou phényle éventuellement substitué et |
| $R^2$, $R^3$, $R^4$ et $R^5$ | sont identiques ou différents et représentent chacun indépendamment des autres, un atome d'hydrogène ou un radical alkyle en $C_1$ à $C_{12}$ qui est éventuellement substitué par des halogènes, des radicaux alcoxy en $C_1$ à $C_{12}$, phényle, phényle substitué, alcoxy($C_1$-$C_{12}$)carbonyle, ou cyano. |